Europäisches Patentamt

European Patent Office

Office européen des brevets

(19)

(11) Numéro de publication: **0 567 579 B1**

# (12) FASCICULE DE BREVET EUROPEEN

(45) Date de publication de fascicule du brevet: **26.07.95**

(51) Int. Cl.⁶: **B01J 19/10**, B01F 3/00, B06B 3/00, B01D 19/00

(21) Numéro de dépôt: **92904795.9**

(22) Date de dépôt: **15.01.92**

(86) Numéro de dépôt internationale :
**PCT/FR92/00029**

(87) Numéro de publication internationale :
**WO 92/12790 (06.08.92 92/21)**

(54) **UNITE MODULAIRE DE REACTEUR ULTRA-SONIOUE TUBULAIRE.**

(30) Priorité: **17.01.91 FR 9100499**

(43) Date de publication de la demande:
**03.11.93 Bulletin 93/44**

(45) Mention de la délivrance du brevet:
**26.07.95 Bulletin 95/30**

(84) Etats contractants désignés:
**AT BE CH DE DK ES FR GB IT LI NL SE**

(56) Documents cités:
**WO-A-82/00260**
**WO-A-82/03795**
**FR-A- 2 354 827**
**US-A- 4 074 152**
**US-A- 4 392 380**

(73) Titulaire: **VAXELAIRE, Philippe**
**6, rue Henri-Jaccaz**
**F-74100 Ville-la-Grand (FR)**

(72) Inventeur: **VAXELAIRE, Philippe**
**6, rue Henri-Jaccaz**
**F-74100 Ville-la-Grand (FR)**

(74) Mandataire: **Ahner, Francis et al**
**CABINET REGIMBEAU**
**26, avenue Kléber**
**F-75008 Paris (FR)**

EP 0 567 579 B1

Rank Xerox (UK) Business Services
(3.10/3.09/3.3.3)

## Description

La présente invention concerne une unité modulaire de réacteur ultrasonique tubulaire ainsi que les diverses associations ces dites unités en vue de former des multiréacteurs adaptés à chaque type d'application particulière.

Dans l'état actuel de la technique on connaît divers dispositifs ultrasoniques destinés à réaliser le traitement des liquides. Il s'agit principalement de dispositifs issus de la technologie du nettoyage par ultrasons et généralement agencés sous forme de cuves métalliques ce faible épaisseur au fond desquelles sont fixés une pluralité d'émetteurs.

La mise en vibration du fond de la cuve induit dans le liquide contenu le phénomène de cavitation dont l'intensité décroit à mesure qu'on s'éloigne de la source, en l'occurence le fond de la cuve.

On connaît également une technique dite à double diaphragmes en opposition, qui, tout comme la technique de nettoyage précitée, consiste à disposer un certain nombre d'émetteurs sur deux plaques ce tôle mises en regard à une distance comprise entre 1,5 et 4 mm, appelée distance de couplage. On fait ensuite passer le liquide à traiter entre las deux plaques émissives.

Une autre configuration utilise une pluralité d'émetteurs axes à la périphérie d'un tube métallique. Cette configuration permet certes ce traiter en passage mais présente les inconvénients de nécessiter un grand nombre d'émetteurs, de ne pas offrir la possibilité de dépasser le niveau d'amplitude couramment obtenu en technique de nettoyage et impose le remplacement complet du système en cas d'usure du tube de traitement en contact avec le produit traité.

Toutes les techniques connues se ramènent en fait a des techniques de nettoyage par ultrasons où les émetteurs sont directement fixés à des diaphragmes métalliques, tubes ou fonds de cuve.

L'état de la technique peut également être illustré par WO-A-82 03795, qui décrit une installation de dégazéification acoustique de liquides, mais en aucun cas ne suggère un réacteur tubulaire ultrasonique fondé sur le principe de la résonnance mécanique en vue de propager les vibrations ultrasoniques de façon cohérente à partir d'un nombre réduit d'émetteurs.

On connait enfin les sondes expérimentales de laboratoire dites sondes ultrasoniques bien connues par exemple sous le nom de Sonifier (BRANSON) ou Vibracell (SONIC'S et MATERIALS). Ce type d'appareils permet de traiter de faibles volumes ou de faibles débits.

La présente invention à pour but de proposer un réacteur ultrasonique tubulaire basé sur le principe de la résonnance mécanique pour propager de façon cohérente à partir d'un nombre d'émetteurs réduit des vibrations ultrasoniques destinées au traitement des fluides traversant ledit réacteur.

Conformément à la présente invention, cet objectif a pu être atteint par la mise au point d'une unité modulaire de réacteur caractérisée en ce qu'elle comprend un corps métallique tubulaire de surface intérieure cylindrique et de section droite circulaire, ouvert à ses deux extrêmités d'alimentation et d'évacuation, en ce que la surface extérieure dudit corps métallique tubulaire présente, au voisinage de la zone nodale, une couronne coaxiale audit tube et faisant saillie radialement, et en ce qu'au moins un convertisseur ultra-sonique est agencé radialement et de façon solidaire de ladite couronne à la périphérie de cette dernière, la fréquence dudit convertisseur étant égale à la fréquence de vibration de ladite couronne et à la fréquence de vibration longitudinale dudit corps métallique tubulaire.

D'autres caractéristiques et avantages de l'objet de la présente invention apparaîtront à la lecture de la description détaillée faite ci-après notamment en référence aux dessins annexés illustrant un certain nombre de modes de réalisation particuliers, dessins sur lesquels :

- la figure 1A représente une vue de côté d'une unité modulaire de réacteur selon l'invention,
- la figure 1B réprésente une vue de face de l'unité modulaire de réacteur de la figure 1A,
- la figure 2 représente un multi-réacteur en ligne comportant plusieurs unités modulaires de réacteur selon l'invention associé à un convertisseur unique,
- la figure 3 représente un multi-réacteur en ligne comportant une pluralité d'unités modulaires de réacteur associées à trois convertisseurs distincts,
- la figure 4 représente un multi-réacteur en ligne comprenant une seule unité modulaire dissymétrique au niveau de la prolongation de la partie tubulaire dudit réacteur,
- la figure 5 représente un autre mode de réalisation d'un multi-réacteur en ligne associant diverses unités modulaires à plusieurs convertisseurs, et
- la figure 6 représente une unité modulaire dont la couronne est rapportée sur le corps métallique tubulaire, par exemple par emmanchement à force,
- la figure 7 représente une unité modulaire utilisant un élément tubulaire intérieur couplé à ladite unité au voisinage des ventres d'amplitude longitudinale (noeud de contrainte).

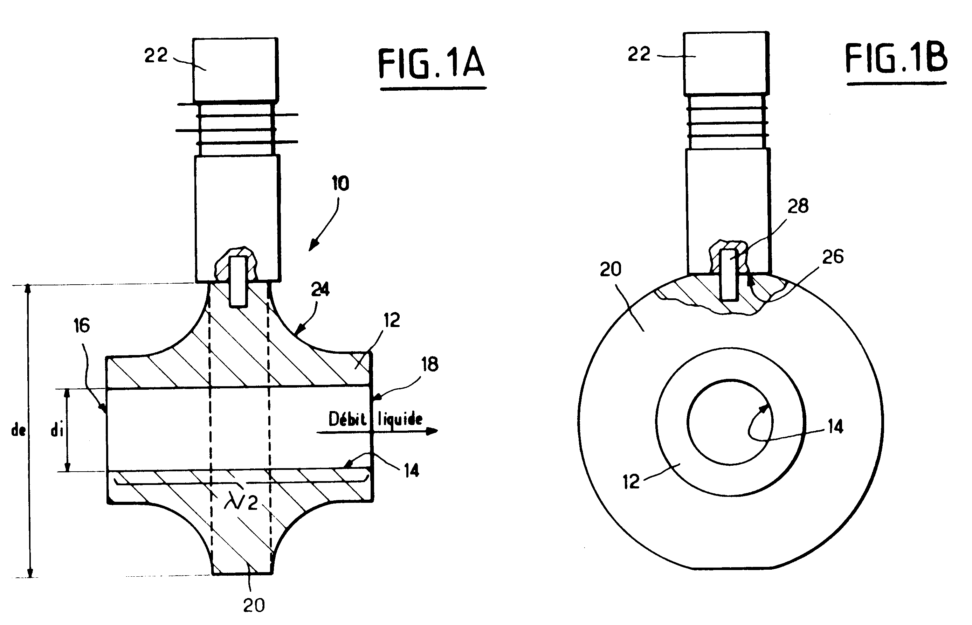

Tel que représenté sur les figures 1A et 1B illustrant respectivement une vue de côté et une vue de face de l'unité modulaire de réacteur 10 selon l'invention, on constate que ladite unité est principalemement constituée de trois éléments caractéristiques essentiels. L'unité 10 comprend tout d'abord un corps métallique tubulaire 12 présentant une surface intérieure 14 cylindrique et de section droite circulaire. Le corps métallique tubulaire 12 est ouvert à ses deux extrêmités, c'est-à-dire respectivement à son extrêmité d'alimentation 16 et à son extrêmité d'évacuation 18. Le flux des matières et/ou des réactifs à traiter s'écoule donc dans le sens de la flèche allant de l'extrêmité 16 vers l'extrêmité 18. Ces deux extrêmités d'alimentation et d'évacuation 16, 18 seront associées à des conduits d'alimentation et d'évacuation eux-mêmes éventuellement munis de pompes de circulation. Le reste de l'installation n'est pas représenté étant donné qu'il fait appel à des organes tout à fait classiques bien connus de l'homme du métier.

L'unité modulaire 10 comprend également sur la surface extérieure du corps métallique tubulaire 12, au voisinage de la zone nodale de cette dernière, une couronne 20 coaxiale audit tube, ladite couronne faisant radialement saillie vers l'extérieur de la surface libre du corps tubulaire 12.

Enfin, l'unité modulaire 10 comprend au moins un convertisseur ultra-sonique 22 qui est agencé radialement et de façon solidaire de ladite couronne 20 à la périphérie de cette dernière. Conformément à la présente invention la fréquence dudit convertisseur 22 est égale à la fréquence de vibration de ladite couronne 20 et à la fréquence de vibration longitudinale du corps métallique tubulaire 12.

Dans la pratique on utilise comme convertisseur ultra-sonique 22 un convertisseur classique, par exemple du type à excitation piezoélectrique. Il peut par exemple être du type "Triplet de Langevin" tel que cela se trouve décrit dans l'ouvrage High Intensity Ultrasonics de B. BROWN et J.E. GOODMAN.

Sur le mode de réalisation particulier décrit et représenté aux figures 1A et 1B, la couronne coaxiale 20 est usinée dans la masse du corps métallique tubulaire 12. Dans ce type de réalisation, la couronne 20 se raccorde à la surface extérieure du corps tubulaire 12 par l'intermédiaire de congés de raccordement 24. On observera que le corps métallique tubulaire 12 présente, sur le mode de réalisation décrit, une longueur égale à une demi-longueur d'onde pour la fréquence que l'on désire utiliser. Il est à noter à ce propos que la fréquence de la vibration ultra-sonique délivrée par l'émetteur ou le convertisseur 22 sera généralement comprise entre 5 et 100 kHz. Dans ce mode de réalisation particulier la longueur du corps métallique tubulaire est rigoureusement égale à une demi longueur d'onde de la fréquence de la vibration ultra-sonique. Il est cependant parfaitement possible, dans le cadre de la présente invention, d'avoir recours à une partie métallique tubulaire de plus grande dimension, se prolongeant par exemple d'un ou des deux côtés de la couronne coaxiale 20 par une longueur égale à un multiple entier de demi longueur d'onde de la fréquence délivrée, la liaison de cette partie métallique avec l'unité modulaire pouvant par exemple être réalisée au niveau des ventres d'amplitude longitudinaux (noeuds de contraintes) au moyen de filetages. emmanchements à force, soudures ou analogue ou bien encore être usinee dans la masse.

On rappellera à ce propos que les diamètres intérieur et extérieur de la couronne 20, le diamètre intérieur de la couronne 20 correspondant par ailleurs au diamètre intérieur du corps métallique tubulaire 12, déterminent la fréquence radiale de cette couronne coaxiale 20. En revanche la longueur du corps métallique tubulaire 12 que l'on choisit en multiple de demi longueur d'onde détermine la fréquence longitudinale de l'ensemble du réacteur.

Dans le mode de réalisation particulier décrit aux figures 1A et 1B on utilise un seul convertisseur ou émetteur 22 qui peut être indifféremment du type électrostrictif, magnétostrictif, électrocapacitif, ou encore plus habituellement piézo-électrique . Le convertisseur 22 est fixé à la périphérie de la couronne 20 de préférence au niveau d'un méplat 26 agencé de manière à assurer une parfaite mise en contact de l'émetteur 22 avec la couronne 20. La liaison mécanique entre ces deux organes est notamment assurée de façon avantageuse par l'intermédiaire d'un goujon ou organe analogue 28.

En ce qui concerne la fréquence de la vibration ultra-sonique délivrée par le convertisseur 22 de l'unité modulaire de réacteur 10 selon l'invention il y a lieu de préciser que les diamètres intérieurs et extérieurs respectivement $d_i$ et $d_e$ de la couronne 20 peuvent être déterminés de façon classique par l'homme du métier en relation avec ladite fréquence de vibration délivrée par l'émetteur. Dans le cas d'une couronne 20 présentant par exemple une épaisseur de l'ordre de 15 mm, les diamètres intérieurs et extérieurs de cette dernière sont déterminés en fonction de la fréquence de vibration F conformément à la relation :

$$F = \frac{C.\ 1{,}08}{\dfrac{d_i + d_e . \pi}{2}}$$

où C représente la vitesse du son, en cm par seconde, associée au metal dans lequel est réalisée ladite couronne 20.

On peut citer par exemple une couronne réalisée en alliage d'aluminium excitée à partir d'un émetteur piézoélectrique 2O kHz ce diamètre 40 mm. Dans ce cas de figure la couronne aura une épaisseur de 30 mm, un diamètre extérieur de 130 mm pour un diamètre intérieur de 42 mm pour respecter la fréquence de 20 kHz délivrée par l'émetteur.

Une telle unité de réacteur fonctionne de la manière suivante. La vibration produite par le ou par les convertisseur(s) 22 provoque la mise en vibration radiale de la couronne 20, qui, en même temps, provoque la mise en vibration longitudinale de la partie tubulaire en multiple de demi longueur d'onde du corps métallique tubulaire 12. La phase de compression de la couronne 20 correspond à la phase de dilatation longitudinale du corps tubulaire 12, et la dilatation de la couronne 20 correspond à la phase de compression longitudinale du corps tubulaire 12. Pour obtenir un tel résultat on rappellera que la couronne coaxiale 20 doit être située au voisinage du plan nodal du corps tubulaire 12. Ainsi, le réacteur selon l'invention utilise la vibration radiale du diamètre intérieur de la couronne 20 pour mettre en cavitation un liquide quelconque à traiter, qui se trouve débité en continu au travers du diamètre intérieur du corps tubulaire métallique 12.

Ainsi, tout liquide, tout gaz, tout colloïde ou encore toute autre matière à traiter doivent être par des moyens classiques non représentés débités au travers du diamètre intérieur 14 du corps métallique tubulaire 12 du réacteur 10.

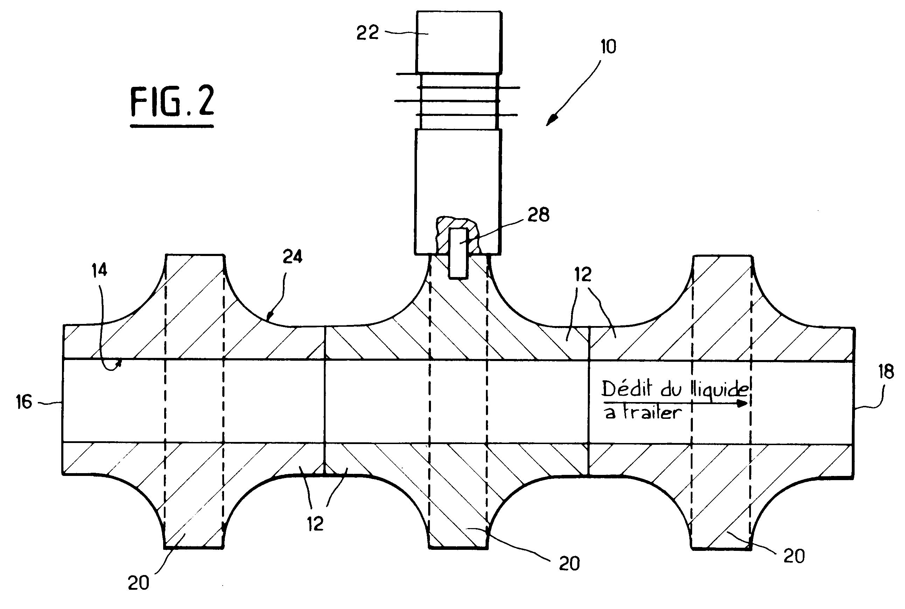

La figure 2 représente un réacteur ultra-sonique qui est obtenu par coupage en série d'une pluralité d'unités modulaires de réacteur 10 tel que représenté aux figures 1A et 1B. Dans le mode de réalisation de la figure 2, on a regroupé trois unités modulaires 10 de manière à former un multi-réacteur en ligne, mais en n'utilisant qu'un seul convertisseur ultra-sonique 22. Ainsi un tel ensemble peut être réalisé monobloc ou par liaisons rigides desdites unités modulaires au niveau des ventres d'amplitude longitudinaux.

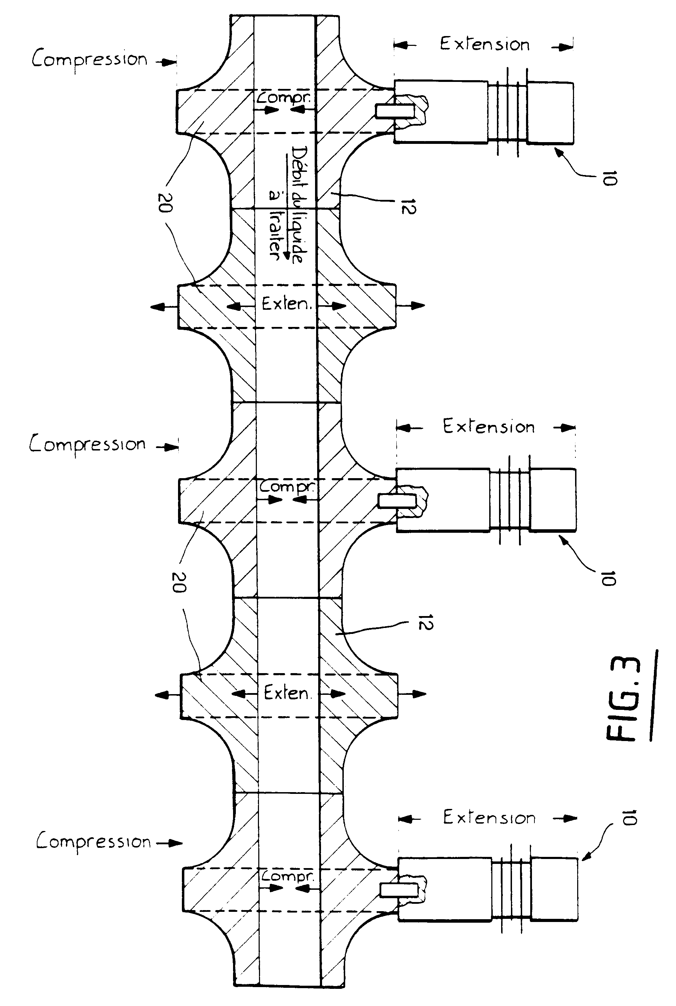

Il est également possible, tel que cela se trouve illustre à la figure 3 de réaliser un multi-réacteur en ligne par association de plusieurs unités modulaires de réacteur 10 couplées à une pluralité de convertisseurs 22. Dans le mode de réalisation particulier décrit, on utilise trois unités modulaires complètes équipées chacune de leur convertisseur 22, ces unités complètes étant disposées aux deux extrêmités et au centre du réacteur tubulaire. On observera qu'entre chacune de ces unités modulaires 10 complètes en demi longueur d'onde, on a interposé un ensemble constitué par un corps métallique tubulaire 12 également en demi longueur d'onde se prolongeant à sa périphérie par une couronne coaxiale 22. Ceci revient également à une association d'unités modulaires de réacteur précédemment décrites dont certaines sont en demi longueur d'onde et d'autres en longueur d'onde entière.

Le fonctionnement de ce type de réacteur en ligne est identique au fonctionnement des unités modulaires précédemment décrites. Dans la même phase du mouvement vibratoire, on observera donc pour les différents éléments associés des états simultanés, de proche en proche, en compression et en dilatation.

Lorsque le réacteur ultra-sonique en ligne comprend plusieurs émetteurs ou convertisseurs ultra-soniques 22, ces derniers peuvent être avantageusement alimentés en parallèle par un même générateur. Il convient cependant de prendre la précaution d'inverser la polarité des émetteurs de rang pair par rapport aux émetteurs de rang impair, ceci dans le but de respecter les inversions de phases qui se produisent à chaque demi longueur d'onde.

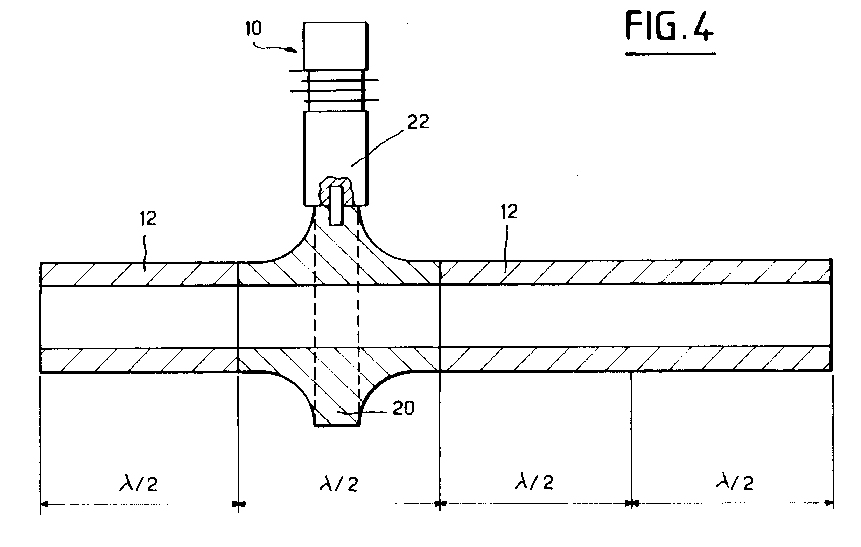

Le mode de réalisation particulier représenté à la figure 4 comprend une couronne radiale 20 qui va exciter en série une longueur de corps métallique tubulaire prolongeant la partie tubulaire de l'élément modulaire central de part et d'autre de ce dernier. On observera que la longueur des tubes de chacun des deux côtés de l'unité modulaire centrale est également réalisée en un multiple entier de demi longueur d'onde. Les dits tubes peuvent par exemple être vissés ou emmanchés à force à l'unité modulaire au niveau des ventres d'amplitude longitudinaux. Il n'est pas nécessaire d'avoir recours à une disposition symétrique. Tel que cela se trouve illustré à la figure 4, il peut être avantageux en fonction des applications particulières envisagées de prévoir une zone tubulaire plus ou moins longue en amont ou en aval de la zone de vibration ultra-sonique intense. Dans la pratique, on aura recours à un multiple de demi longueur d'onde généralement compris entre 1 et 10, multiple choisi en fonction du matériau dans lequel on réalise le réacteur, en fonction des produits à traiter et bien sûr, de la nature et des conditions de la réaction mise en jeu au sein du réacteur. Il peut par exemple être avantageux, comme cela se trouve illustré sur la figure 4, de prévoir une entrée d'alimentation relativement proche de la zone intense de cavitation, ce qui permet de communiquer rapidement l'énergie nécessaire à l'initiation de la réaction qui peut se prolonger dans la partie tubulaire avale du réacteur, i.e. dans une zone soumise à une cavitation ultra-sonique de moins grande puissance et de moins grande amplitude.

L'homme du métier choisira, en fonction de l'application particulière, la longueur adaptée dudit corps métallique tubulaire 12 en respectant toutefois une longueur égale à un multiple entier de demi longueurs d'onde de la fréquence de la vibration ultra-sonique délivrée par le ou les convertisseurs 22.

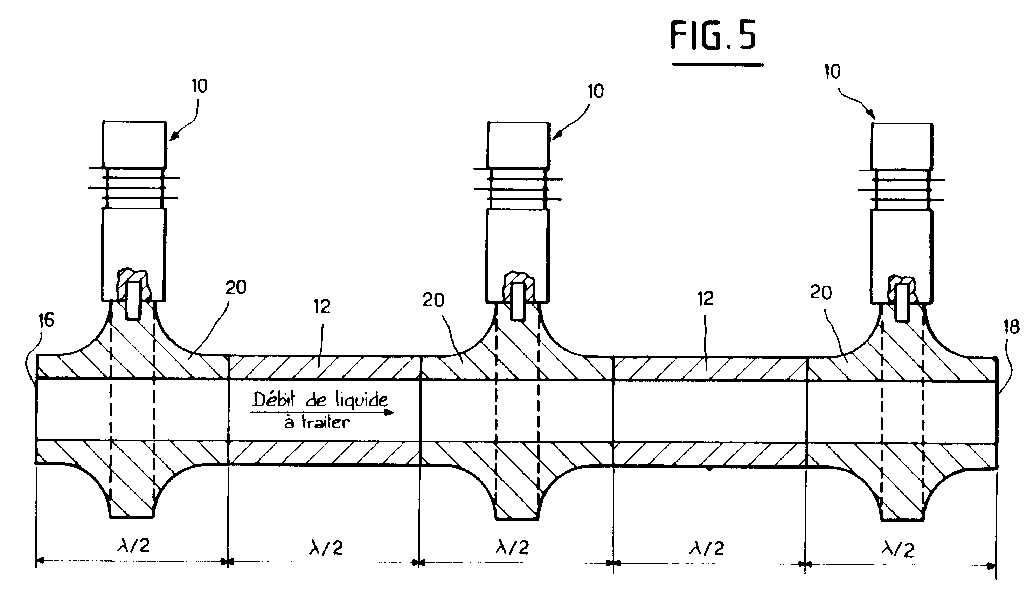

La figure 5 illustre un dernier mode de réalisation dans lequel on a recours à plusieurs couronnes radiales 20 qui vont exciter en serie une longueur de tube qui, en l'espèce est en demi longueur d'onde, mais qui bien entendu peut également être constitué par de plus grandes longueurs de tubes réalisées en multiple entier de demi longueurs d'onde. Le principe de fonctionnement reste rigoureusement identique.

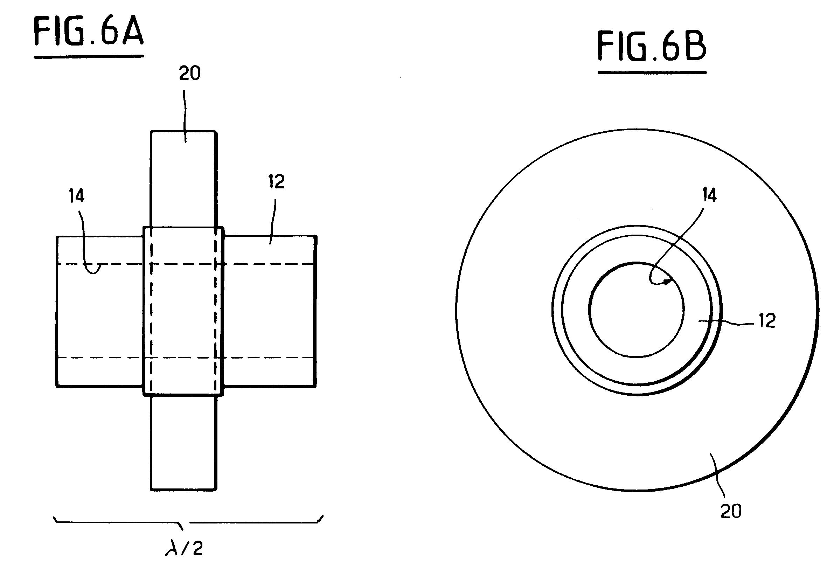

La figure 6 représente une variante d'une unité modulaire de réacteur selon l'invention. Conformément à cette variante, au lieu d'être usinée dans la masse, la couronne coaxiale 20 et le corps tubulaire métallique 12 sont réalisés séparément. Ceci permet de choisir un matériau d'excellente propriété accoustique tel qu'un alliage d'aluminium et/ou de titane, pour la fabrication de la couronne 20, et d'avoir recours à un autre matériau présentant une plus grande résistance à l'érosion de cavitation ultra-sonique pour la fabrication de la partie tubulaire métallique 12 à l'intérieur de laquelle circule le liquide à traiter. On pourra ainsi réaliser la partie tubulaire 12 en un acier spécial tel qu'un acier inoxydable. En outre, la surface intérieure 14 du réacteur tubulaire sera avantageusement munie d'une couche de protection contre l'érosion par cavitation, par exemple d'une couche céramique, métallique dure ou encore céramométallique. Dans ce mode de réalisation de la figure 6, la couronne est rendue solidaire de la partie tubulaire par un moyen mécanique approprié assurant une liaison solide et homogène. Une telle liaison rigide absolument indispensable pour réaliser l'unité modulaire de reacteur selon l'invention, peut par exemple être obtenue par des moyens mécaniques tels qu'un vissage, un blocage, ou encore un emmanchement à force. On peut également avoir recours à d'autres moyens non mécaniques, tels que des collages, soudages ou analogues.

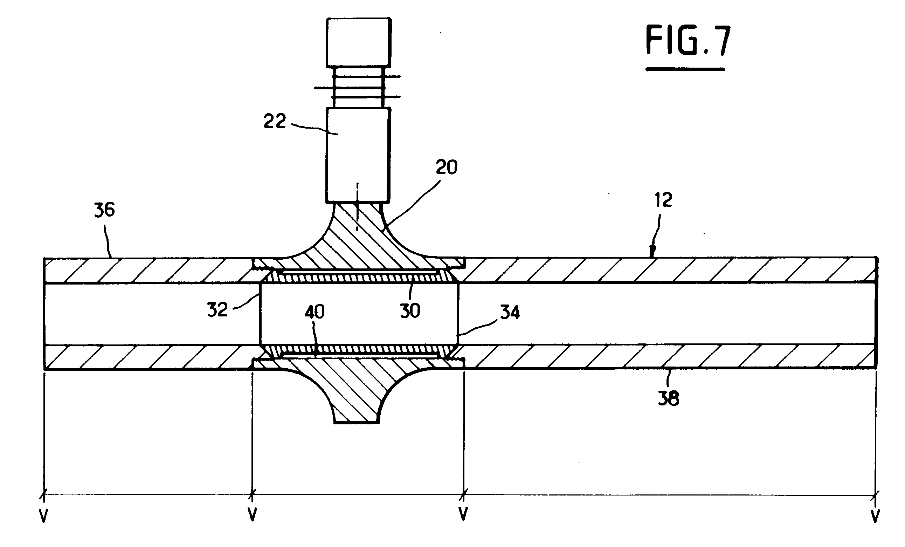

- La figure 7 représente une autre variante d'une unité modulaire de réacteur selon l'invention.

Conformément à cette variante, l'unité modulaire de réacteur est agencée de manière à recevoir à l'intérieur un élément tubulaire 30 accordé à la fréquence de l'émetteur 22, vibrant à partir du mode axial par serrage de ses deux extrémités 32,34 au voisinage des ventres d'amplitude (noeuds de contraintes) longitudinale de ladite unité.

Dans ce mode de réalisation, ledit élément tubulaire 30 est serré entre deux pièces elles-mêmes accordées à la fréquence de l'émetteur et pouvant être, par exemple, des corps métalliques tubulaires 36 et 38 tels que décrits sur la figure 4.

L'élément tubulaire rapporté 30, sera avantageusement agencé avec des portées soignées (i.e. 45°) pour assurer un parfait centrage et un bon couplage avec les éléments 36 et 38 le serrant à chacune de ses extrémités 32 et 34. On notera, tel que décrit sur la figure 7, un jeu 40 avec le diamètre intérieur de l'unité modulaire permettant les libres expansion et compression radiales de l'élément tubulaire 30 à l'intérieur duquel circule le fluide à traiter.

Ainsi, les unités modulaires de réacteur selon l'invention, peuvent être utilisées pour le traitement de tous types de liquide, gaz, colloïde, etc. débitant de façon naturelle ou forcée à travers le diamètre intérieur des réacteurs. Il convient également de noter qu'un flux de liquide extérieur pourraît être simultanément traité par la cavitation provoqué par la vibration extérieure des couronnes 20, c'est-à-dire que l'on pourrait traiter simultanément deux flux de liquides différents, l'un passant à l'intérieur du réacteur, l'autre s'écoulant à la périphérie extérieure.

Les applications industrielles de ce type de réacteurs sont nombreuses : on citera par exemple à titre d'illustration les applications suivantes :
- activations de réactions chimiques (Grignard, Barbier, etc.)
- désagglomération et lavage de poudres métalliques, céramiques ou minérales
- traitement de minerals
- mouillage et dispersions de pigments, colorants
- dissolution accélérée de gaz
- dégazage
- homogénéisation de produits
- nettoyage de fils
- émulsification, etc.

**Revendications**

1. Unité modulaire de réacteur pour le traitement ultrasonique en continu de matières et/ou de réactifs, caractérisé en ce qu'elle comprend un corps métallique tubulaire (12) de surface intérieure (14) cylindrique et de section droite circulaire, ouvert à ses deux extrémités d'alimentation (26) et d'évacuation (18), en ce que la surface extérieure dudit corps métallique tubulaire présente, au voisinage de la zone nodale, une couronne (20) coaxiale audit tube et faisant saillie radialement, et en ce qu'au moins un convertisseur ultrasonique (22) est agencé radialement et de façon solidaire de ladite couronne à la périphérie de cette dernière, et en ce que la longueur du corps métallique tubulaire (12) est égale à un multiple entier de demi-longueur d'onde de la fréquence de vibration ultrasonique délivrée par le convertisseur (22), les diamètres intérieur et extérieur de la couronne (20) étant déterminés de manière que la, fréquence de vibration de ladite couronne soit également à la même fréquence de vibration ultrasonique délivrée par le convertisseur (22).

2. Unité modulaire de réacteur selon la revendication 1, caractérisé en ce que ledit convertisseur ultrasonique (22) est fixé à la périphérie de ladite couronne (20) au niveau d'un méplat agencé de manière à assurer un parfait couplage du convertisseur (26) avec la couronne, la liaison mécanique étant assurée par l'intermédiaire d'un goujon (28) ou analogue.

3. Unité modulaire de réacteur selon l'une des revendications 1 et 2, caractérisée en ce que ladite couronne (20) est usinée dans la masse métallique du corps tubulaire (12).

4. Unité modulaire de réacteur selon l'une des revendications 1 et 2, caractérisée en ce que ladite couronne (20) est rapportée sur ledit corps métallique tubulaire (12) par tout moyen assurant une liaison rigide.

5. Unité modulaire de réacteur selon la revendication 4, caractérisée en ce que ladite couronne (20) est réalisée en un métal présentant de bonnes propriétés acoustiques, tel qu'un alliage d'aluminium et/ou de titane, et en ce que ledit corps métallique tubulaire (12) est réalisé en un métal doté d'une bonne résistance à l'érosion par cavitation, tel que l'acier inoxydable.

6. Unité modulaire de réacteur selon l'une des revendications 1 à 5, caractérisée en ce que la surface intérieure (14) du réacteur tubulaire est munie d'une couche de protection contre l'érosion par cavitation du réacteur, ladite couche étant constituée par un revêtement céramique, métallique dur ou céramométallique.

7. Unité modulaire de réacteur selon l'une des revendications 1 à 4, caractérisée en ce qu'un élément tubulaire (30) est rapporté à l'intérieur de ladite unité par tout moyen de serrage, au voisinage des ventres d'amplitude longitudinale de ladite unité.

8. Réacteur ultrasonique, caractérisé en ce qu'il est réalisé par couplage en série d'une pluralité d'unités modulaires de réacteur (10) selon l'une des revendications 1 à 7, de manière à créer un multi-réacteur en ligne.

9. Réacteur ultrasonique selon la revendication 8, caractérisé en ce qu'il comprend plusieurs convertisseurs (22) alimentés en parallèle par le même générateur.

## Claims

1. Modular reactor unit for continuous ultrasonic treatment of materials and/or reagents characterized in that it comprises a tubular metal body (12) having a cylindrical interior surface (14) and a circular transverse cross-section open at a feed end (26) and a discharge end (18), in that the exterior surface of said tubular metal body has in the vicinity of the nodal zone a radially projecting collar (20) coaxial with said tube, and in that at least one ultrasonic converter (22) is disposed radially and attached to said collar at its periphery, and in that the length of said tubular metal body (12) is equal to an integer multiple of half the wavelength at the frequency of ultrasonic vibration emitted by said converter (22), the inside and outside diameters of the collar (20) being such that the frequency of vibration of said collar is also equal to the frequency of ultrasonic vibration emitted by the converter (22).

2. Modular reactor unit according to claim 1 characterized in that said ultrasonic converter (22) is fixed to the periphery of said collar (20) at a flat adapted to ensure perfect coupling of the converter (26) and the collar, the mechanical coupling being provided by a pin (28) or the like.

3. Modular reactor unit according to claim 1 or claim 2 characterized in that said collar (20) is machined in one piece with the tubular metal body (12).

4. Modular reactor unit according to claim 1 or claim 2 characterized in that said collar (20) is attached to said tubular metal body (12) by any means providing a rigid coupling.

5. Modular reactor unit according to claim 4 characterized in that said collar (20) is made from a metal having good acoustical properties such as aluminum and/or titanium alloy and in that said tubular metal body (12) is made from a metal having a good resistance to erosion caused by cavitation such as stainless steel.

6. Modular reactor unit according to any one of claims 1 to 5 characterized in that the interior surface (14) of the tubular reactor has a ceramic, hard metal or combined ceramic/metal coating to protect it against erosion by cavitation.

7. Modular reactor unit according to any one of claims 1 to 4 characterized in that a tubular member (30) is attached to the interior of said unit by clamping means in the vicinity of longitudinal maximum amplitude regions of said unit.

8. Ultrasonic reactor characterized in that it comprises a series coupling of a plurality of modular reactor units (10) according to any one claims 1 to 7 to create an in-line multireactor.

9. Ultrasonic reactor according to claim 8 characterized in that it comprises a plurality of converters (22) fed in parallel by the same generator.

**Patentansprüche**

1. Modulare Reaktoreinheit für die kontinuierliche Ultraschallbehandlung von Materialien und/oder Reagenzien, dadurch gekennzeichnet, daß sie einen rohrförmigen, metallischen Körper (12) mit zylindrischer Innenfläche (14) und kreisförmigem Querschnitt aufweist, an seinen beiden Enden für die Versorgung (26) und für die Ableitung (18) geöffnet, daß die Außenfläche des rohrförmigen, metallischen Körpers, in der Umgebung der Knotenzone, einen Kranz (20), der koaxial zu dem Rohr ist und radial hervorsteht, zeigt und daß wenigstens ein Ultraschallwandler (22) radial und an dem Kranz festgelegt am Außenumfang des letzteren angeordnet ist und daß die Länge des rohrförmigen, metallischen Körpers (12) gleich einem ganzzahligen Vielfachen der Halbwellenlänge der Ultraschall-Schwingungsfrequenz ist, die von dem Wandler (22) geliefert wird, wobei der Innen- und Außendurchmesser des Kranzes (20) derart bestimmt sind, daß die Schwingungsfrequenz des Kranzes gleich derselben Ultraschall-Schwingungsfrequenz ist, die vom dem Wandler (22) geliefert wird.

2. Modulare Reaktoreinheit nach Anspruch 1, dadurch gekennzeichnet, daß der Ultraschallwandler (22) an dem Außenumfang des Kranzes (20) auf der Höhe einer Abflachung befestigt ist, die derart angeordnet ist, daß eine perfekte Kopplung des Wandlers (22) mit dem Kranz sichergestellt ist, wobei die mechanische Verbindung durch Zwischenschaltung eines Stiftbolzens (28) oder dergleichen gewährleistet ist.

3. Modulare Reaktoreinheit nach einem der Ansprüche 1 und 2, dadurch gekennzeichnet, daß der Kranz (20) in der metallischen Masse des rohrförmigen Körpers (12) gefertigt ist.

4. Modulare Reaktoreinheit nach einem der Ansprüche 1 und 2, dadurch gekennzeichnet, daß der Kranz (20) über irgendein Mittel, das eine starre Verbindung sicherstellt, an den rohrfömigen, metallischen Körper (12) angefügt ist.

5. Modulare Reaktoreinheit nach Anspruch 4, dadurch gekennzeichnet, daß der Kranz (20) aus einem Metall hergestellt ist, das gute akustische Eigenschaften zeigt, so wie einer Aluminium- und/oder Titanlegierung, und daß der rohrförmige metallische Körper (12) aus einem dotierten Metall mit einem guten Widerstand gegen die Errosion durch Kavitation hergestellt ist, so wie rostfreiem Stahl.

6. Modulare Reaktoreinheit nach einem der Ansprüche 1 bis 5, dadurch gekennzeichnet, daß die Innenfläche (14) des rohrförmigen Reaktors mit einer Schutzschicht gegen die Erosion durch Kavitation des Reaktors versehen ist, wobei die Schicht aus einem keramischen, hartmetallischen oder keramometallischen Überzug gebildet ist.

7. Modulare Reaktoreinheit nach einem der Ansprüche 1 bis 4, dadurch gekennzeichnet, daß ein rohrförmiges Element (30) in das Innere der Einheit durch eine Verriegelungseinrichtung eingefügt ist, in der Umgebung der Bäuche der Längsamplitude der Einheit.

8. Ultraschallreaktor, dadurch gekennzeichnet, daß er durch Serienkopplung einer Vielzahl von modularen Reaktoreinheiten (10) nach einem der Ansprüche 1 bis 7 hergestellt ist, derart, daß ein Reihen-Vielfachreaktor erzeugt wird.

9. Ultraschallreaktor nach Anspruch 8, dadurch gekennzeichnet, daß er mehrere Wandler (22) aufweist, die parallel durch denselben Generator versorgt werden.

FIG.1A

FIG.1B

Débit liquide

EP 0 567 579 B1

FIG.2

Dédit du liquide à traiter

FIG.3

## FIG.4

## FIG.5

Débit de liquide à traiter

10 10 10

12 12

20 20 20

18 16

λ/2 λ/2 λ/2 λ/2 λ/2

FIG.6A

20

14

12

λ/2

FIG.6B

14

12

20

EP 0 567 579 B1

FIG.7

EP 0 567 579 B1